# EUROPÄISCHE PATENTSCHRIFT

(11) **EP 0 560 469 B1**
(45) Veröffentlichungstag und Bekanntmachung des Hinweises auf die Patenterteilung: **28.05.1997**
(21) Anmeldenummer: 93250076.2
(22) Anmeldetag: 10.03.1993
(51) Int. Cl.: G01N 27/327, C12M 1/40, C12Q 1/00

(54) **Sandwich-Membran für Biosensoren und deren Verwendung**
Sandwich-membrane for biosensors and use thereof
Membrane de type sandwich pour des biosenseurs et son utilisation

(30) Priorität: 11.03.1992 DE 4208186
(43) Veröffentlichungstag der Anmeldung: 15.09.1993
(73) Patentinhaber: BST BIO SENSOR TECHNOLOGIE GmbH, D-13156 Berlin (DE)
(72) Erfinder: Pfeiffer, Dorothea, Dr. (Geb. Seidel), O-1100 Berlin (DE); Scheller, Frieder, Prof. Dr., O-1297 Zepernick (DE); Klimes, Norbert, O-1100 Berlin (DE); Nentwig, Jürgen, O-1144 Berlin (DE)
(74) Vertreter: Baumbach, Friedrich, Dr.

(56) Entgegenhaltungen:
- EP-A- 0 079 502
- EP-A- 0 216 577
- EP-A- 0 396 156
- DE-A- 3 824 258
- DE-A- 4 104 776
- GB-A- 2 197 486
- GB-A- 2 209 836
- JOURNAL OF ANALYTICAL CHEMISTRY OF USSR Bd. 45, Nr. 7 , Juli 1990 , NEW YORK US Seiten 1033 - 1038 L. P. KUZNETSOVA ET AL. 'Characteristics of enzyme-containing membranes for biosensors.'

## Beschreibung

Die Erfindung betrifft eine Sandwich-Membran für Biosensoren, die auch für die Bestimmung von Substanzen in unvorbehandelten Proben geeignet ist, sowie deren Verwendung.
Anwendungsgebiete der Erfindung sind neben der medizinischen Diagnostik die Lebensmittelindustrie, die Fermentationstechnik, die Biotechnologie sowie der Umweltschutz.

Es sind mehrere Möglichkeiten bekannt, Bio-, speziell Enzymelektroden zur Bestimmung von Substratkonzentrationen zu verwenden.
1. Die Sauerstoffverbrauchsmessung bei Oxidase-Enzymelektroden oder mikrobiellen Sensoren.
   Diese aus Appl. Microbiol. Biotechnol. 28 (1988) 316-318 (Riedel et al.) bekannte Methode hat den Vorteil, daß elektrochemisch aktive Substanzen wie Ascorbinsäure, Harnsäure oder Paracetamol nicht störend wirken. Nachteilig ist jedoch, daß eine Übereinstimmung der Sauerstoffkonzentration in der Meßprobe und im Verdünnungspuffer vorhanden sein muß, da die Elektrode nur die Summe von O₂-Änderungen aus der Substratumsetzung und O₂-Konzentration in der Meßlösung erfaßt.
   Man hat versucht, die daraus resultierenden Störungen durch eine Differenzmessung mit der Enzymelektrode und enzymfreier Elektrode zu beseitigen (z.B. S.J. Updike/G.P. Hicks, Nature 214 (1967) 986), was aber den Nachteil hat, daß ein aufwendiger Abgleich beider Elektroden erforderlich ist. Auch die Luftsättigung des Probestroms vor der Messung durch Bildung eines Aerosols (DD 279262, Riedel et.al.) oder Lufteinleiten unmittelbar in die Meßlösung vor der Enzymelektrode (DD 101229, Reitnauer et.al.) hat nicht voll befriedigen können.
   Ein Nachteil insbesondere von DD 101229 besteht in einer die Messung beeinflussende Schlammbildung.
2. Die Messung durch H₂O₂-Anzeige
   Bei dieser Methode (z.B. G.G. Guilbault/G. Lubrano, ACA 64 (1973) 439) ist das Meßsignal in gewissen Grenzen vom O₂-Gehalt der Probe unabhängig. Allerdings liefern alle Substanzen die bei +600 mV oxidierbar sind, einen Beitrag zum Meßsignal, wodurch falsch hohe Analysenwerte entstehen können.
   Man hat versucht, diese Störungen durch eine Differenzmessung wie bei 1. zu vermeiden (D. Thevenot et.al., Anal. Chem. 51 (1979) 96), was aber die gleichen Nachteile wie oben beschrieben hat. Aus US 3979274 ist eine H₂O₂-selektive Membran bekannt, wobei geladene Gruppen oder kleine Poren die Permeation anionischer Störsubstanzen unterdrücken. Allerdings werden falsch hohe Meßwerte durch Paracetamol nicht vollständig ausgeschaltet (Clin. Chem. 28/4 726 (1982)). Weiterhin ist nachteilig, daß der Meßvorgang verzögert wird und die Methode bei Einsatz der H₂O₂-selektiven Membran signifikant unempfindlicher ist.
3. Messung mittels Mediatoren
   Alternativ zum natürlichen Cosubstrat kann Sauerstoff durch künstliche Elektronenakzeptoren substituiert werden, womit prinzipiell die Nachteile der Sauerstoff-Verbrauchsmessung sowie H₂O₂-Detektion umgangen werden können.
   Allerdings ist die Herstellung definierter und vor allem stabiler Mediator-modifizierter Enzymsensorenfür den mehr als einmaligen Einsatz zur Substratananlyse schwierig und aufwendig. Die Ansprechzeiten solcher Sensoren liegen oft sehr hoch (3 bis 5 Minuten) und die Gegenwart von Luftsauerstoff ruft Störungen im niedrigen Meßbereich hervor (W. Müllen et.al.: Analyst. 110 (1985) 952, G. Geppert/L. Asperger: Bioelectrochem. Bioenerg. 17(1987) 399).
4. Erweiterung des Meßbereichs
   Bei der H₂O₂-Messung ist die Erweiterung des Meßbereichs durch strukturierte Membranen bekannt (DD 297713, Scheller et.al.). Hierbei treten aber nach wie vor die oben geschilderten Nachteile der Substratanalyse über die H₂O₂-Detektion auf. Für eine Analyse auf der Basis der O₂-Detektion ist die in DD 297713 beschriebene Anordnung nicht geeignet, da bei der kathodischen Reduktion Störungen auftreten.
   Aus DE-A-41 04 776 ist zwar bereits eine Sandwich-Enzymmembran für Enzymelektroden und -optoden zur Bestimmung von Substraten bekannt. Jedoch tritt bei Anwendung dieser Membran eine Beeinflussung der Analyse durch den Sauerstoff in der Meßlösung auf.

Die Aufgabe der Erfindung besteht darin, die Nachteile der bekannten Enzymelektroden mit O₂ -bzw. H₂O₂-Anzeige auszuschalten und eine Bio-Membran zu entwickeln, die störungsfrei auch in unvorbehandelten Meßproben unterschiedlicher O₂-Konzentration anwendbar ist. Die Bio-Membran soll auch in Medien einsetzbar sein, die elektrochemisch interferierende Substanzen enthalten.

Die Erfindung wird durch eine Sandwich-Membran gemäß Anspruch 1 und deren Verwendung gemäß Anspruch 12 realisiert, die entsprechenden Unteransprüche 2-11 und 13 sind vorteilhafte Ausführungen.
Die erfindungsgemäße Sandwich-Membran ist nachfolgend schematisch dargestellt. 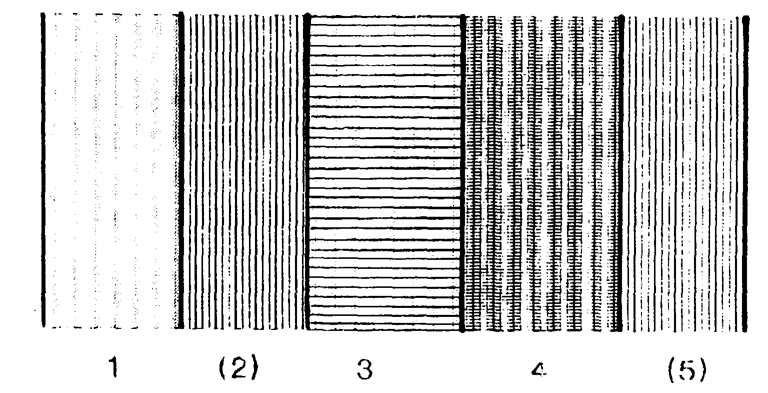
1 (2) 3 4 (5)
1 Gas-selektive Schicht
2 Dialyse-Schicht
3 Biomaterial-Schicht
4 Mikrostrukturierte Schicht
5 Dialyse-Schicht

Die Schichten 1,3 und 4 bilden die notwendigen Bestandteile der Membran. Gegebenenfalls wird zwischen den Schichten 1 und 3 und/oder als Abdeckung zur Meßlösung eine Dialyseschicht (2 bzw. 5)angeordnet.
Die Herstellung der erfindungsgemäßen Sandwich-Membran wird wie folgt realisiert:
Eine acetonische Polyurethanlösung wird mit den entsprechenden Biopartikeln wie Enzymen, Mikroorganismen oder Gewebezellen gemischt. Diese Suspension wird in flüssiger Form auf die als Unterlage fungierende gas-selektive Schicht (1) getropft und bei Zimmertemperatur getrocknet. Die Schicht (1) besteht vorzugsweise aus Polyethylen oder Teflon.
Als Unterlage kann neben der gas-selektiven Schicht (1) auch eine Dialyseschicht (2)fungieren. Sie besteht vorzugsweise aus Cellulose oder Celluloseacetat und wird an der Schicht (1) mechanisch per Halterung fixiert.
Mit dem Abschluß des Trocknungsprozesses ist die gebildete Biomaterialschicht (3) funktionsfähig. Sie wird anschließend mit der mikrostrukturierten Schicht (4) abgedeckt und mechanisch fixiert bzw. es wird zusätzlich noch eine weitere Dialyse-Schicht (5) aufgebracht und dann mechanisch fixiert. Die mikrostrukturierte Schicht (4) besteht vorzugsweise aus einer oder mehreren Polycarbonat-, Polysiloxan-, Polyurethan-, Polysulfonat- oder Polyterephthalteilschichten.
Eine besondere Vorbereitung der Schichten vor der Anhaftung gibt es nicht, abgesehen von dem Aufbringen der Biomaterialschicht auf ihre Unterlage. Das Zusammenhalten aller anderen Schichten erfolgt mechanisch durch Halterungsringe. Zur Messung wird die Elektrode mit der Membran verbunden und dann in eine stehende gerührte oder in eine fließende Lösung eingetaucht. Am anderen Ende ist die Elektrode mit einer Spannungsquelle verbunden (Potentiostat).

Wesentlich für die Funktion der erfindungsgemäßen Membran ist, daß durch die hohe Sauerstoff-Kapazität der mikrostrukturierten Schicht innerhalb der Sandwich-Membran ein sehr großes Cofaktor-Reservoir geschaffen wird. Überraschend ist vor allem, daß die O₂-Kapazität ausreicht, um die Substratanalyse unabhängig vom O₂-Gehalt der zu analysierenden Meßprobe richtig durchzuführen und daß die Analyse durch den Sauerstoff in der Meßlösung nicht beeinflußt wird.

Mit der neuen Membran werden die Mängel des Standes der Technik, insbesondere die Störanfälligkeit durch potentiell interferierende Substanzen sowie die sonst erforderliche Differenzmessung oder Vorbehandlung der Meßprobe mit einem Luftstrom beseitigt. Während die Detektion der Sauerstoff-Reduktion bei -600 mV und die Anwendung einer Dialyseschicht-Enzymschicht-Dialyseschicht-Sandwich-Membran Falschmessungen verursacht, ermöglicht die Abdeckung der Elektrode mit einer gas-selektiven Schicht gemäß der Erfindung die richtige Erfassung der zu analysierenden Substanzen. Der gegebenenfalls erfolgende Einsatz einer weiteren Dialyseschicht zwischen gas-selektiver und Biomaterial-Schicht dient der besseren Haftung der Biomembran.
Somit sind mit der neuen Membran auch störungsfreie Messungen von unvorbehandelten Proben möglich. Weiterhin ist wesentlich, daß die durch die oxidativen Vorgänge in der Membran verbrauchte Sauerstoffkapazität nach abgeschlossener Meßwerterfassung mittels Spülung mit einer luftgesättigten Pufferlösung wieder regeneriert werden kann.

Ein vorteilhafter Einsatz der Membran ist insbesondere für Analysen von Urin, Blut, Fermentationslösungen und Lebensmittelproben sowie Abwässern gegeben.

Die Erfindung soll nachfolgend durch die Ausführungsbeispiele näher erläutert werden.

### Beispiel 1

### Analyse des Urin-Glucosegehaltes mittels Enzymelektrode auf der Basis einer Glucoseoxidase-Sandwichmembran

2 µl einer 5%igen Polyurethanlösung in Aceton, die eine Aktivität von 5 U Glucoseoxidase (GOD) beinhalten, werden auf eine 15 µm dicke Cellulosemembran getropft und im getrockneten Zustand mit einer Kapillarmembran aus Polycarbonat vollständig bedeckt sowie einer zweiten Cellulosemembranm die mit der Enzymunterlage identisch ist, abgedeckt.
Die Kapillarporenmembran (Oxyphen GmbH, Dresden) besitzt eine Dicke von 20 µm und eine Porengröße von 0,16 µm. Eine Pt-Ag/AgCl-Elektrode, die mit einer 15 µm dicken Polyethylenmembran (Metra Radebeul GmbH) bedeckt ist, wird mit der GOD-Membran bedeckt und auf -600 mV polarisiert.

Anschließend wird der Glucosesensor mit der Kapillarmembran zur Lösungsseite hin in ein Fließsystem gebracht.
Der kontinuierlich fließende Pufferstrom (0,1 M Phosphatpuffer pH 7,0; 0,2 ml/min) wird durch das Ansaugen von jeweils 60 µl 5, 10, 15 und 20 mM Glucoselösung unterbrochen. Über die Erfassung des differenzierten Stromsignals dI/dt mittels Bandschreiber wird so die Kalibrierung des Sensors vorgenommen. Danach kann mit 60 µl unverdünntem, unvorbehandeltem Urin innerhalb von 15 sec dessen Glucosekonzentration in einem Bereich von 0,5 bis 25 mM ermittelt werden, indem die Urinprobe vor die Dialyseschicht gepumpt wird.

### Beispiel 2

### Bestimmung der Lactatkonzentration in unvorbehandeltem Vollblut

2 µl einer 5%igen Polyurethanlösung, die 2,5 U Lactatoxidase (LOD) beinhalten, werden in gleicher Weise wie in Beispiel 1 auf die Cellulosemembran getropft und nach Abtrocknung mit einer Polycarbonat-Kapillarporenmembran (Porengröße 0,06 µm) und einer Schichtdicke von 25 µm vollständig bedeckt sowie einer der Unterlage identischen Cellulosemembran abgedeckt.
Die Pt-Ag/AgCl-Elektrode wird mit einer Teflonmembran (Schichtdicke 10 µm) überzogen, weiterhin mit der oben beschriebenen LOD-Sandwichmembran gekoppelt und auf -600 mV polarisiert.
Der Lactatsensor wird in ein Fließsystem gebracht. Eine Rollenpumpe garantiert den definierten Pufferfluß (0,1 M Phosphatpuffer pH 7,0 + 0,5 ml/l Heparin) von 0,1 ml/min.
Die Kalibrierung des Lactatsensors wird mit jeweils 30 µl 5, 10, 15, 20 mM Lactat über die Detektion des differenzierten Stroms dI/dt und Aufzeichnung per Bandschreiber vorgenommen.
Durch Vermessen von 30 µl unvorbehandeltem Vollblut unmittelbar nach der Blutentnahme liegt das Meßergebnis innerhalb von 15 sec mit einer seriellen Impräzision von 5% vor. Der Meßbereich erstreckt sich über eine Konzentration von 0,2 - 20 mM Lactat.

### Beispiel 3

### Analyse der Glucosekonzentration in Fermentationslösungen

2 µl einer 5%igen Polyurethanlösung, die eine Aktivität von 5 U GOD beinhalten, werden auf eine Teflonmembran getropft. Nach der Trocknung der Enzymschicht wird diese vollständig mit einer Kapillarporenmembran mit einem Porendurchmesser von 0,17 µm bedeckt und mit einer Dialysemembran abgedeckt.

Das Sandwich-Membransystem wird mit der Teflonmembran zur Elektrode hin an eine O₂-Elektrode (Metra Radebeul GmbH) gekoppelt (polarisiert auf -600 mV), die an einen amperometrischen Detektor angeschlossen wird.
Der Glucosesensor wird in ein Fließsystem mit einem definierten Pufferfluß von 0,2 ml/min gebracht. Als Systemlösung wird ein Phosphatpuffer verwendet (0,1 M, pH 7,0). Die Kalibrierung des Sensors erfolgt mit dem Vermessen von jeweils 60 µl 10, 20 und 30 mM Glucoselösung. Die differenzierten Stromwerte dI/dt werden mittels Bandschreiber aufgezeichnet, und anschließend wird eine Kalibrationskurve erstellt. 60 µl Fermentationslösung können nach Kalibrierung des Meßsystems innerhalb von 15 sec vermessen werden, wobei sich der Meßbereich über eine Konzentration von 0,25 bis 30 mmol/l Glucose erstreckt.

## Patentansprüche

1. Sandwich-Membran für Biosensoren, gekennzeichnet durch
- eine Biomaterialschicht, die zum Sensor mit einer
- gas-selektiven Schicht abgegrenzt ist und die mit einer
- zur Meßlösung gewandten mikrostrukturierten Schicht abgedeckt ist, deren Permeabilität für verschiedene Substrate/Cosubstrate inhomogen ist,
wobei sich zwischen der gas-selektiven Schicht und der Biomaterialschicht bzw. auf der Meßlösung gewandten Seite der mikrostrukturierten Schicht gegebenfalls
- Dialyseschichten befinden.

2. Sandwich-Membran nach Anspruch 1, dadurch gekennzeichnet, daß die Biomaterialschicht aus einem polymeren Material und darin verteilten Biopartikeln besteht.

3. Sandwich-Membran nach den Ansprüchen 1 und 2, dadurch gekennzeichnet, daß das polymere Material Polyurethan, Polyalginat oder Gelatine ist.

4. Sandwich-Membran nach den Ansprüchen 1 bis 3, dadurch gekennzeichnet, daß das polymere Material mit den darin verteilten Biopartikeln durch bi- oder polyfunktionelle Reagenzien vernetzt ist.

5. Sandwich-Membran nach den Ansprüchen 1 und 2, dadurch gekennzeichnet, daß die Biopartikel bevorzugt aus einem Enzym oder mehreren Enzymen, Mikroorganismen oder Gewebezellen bestehen.

6. Sandwich-Membran nach Anspruch 1, dadurch gekennzeichnet, daß die gas-selektive Schicht aus Polyethylen, Polypropylen oder Teflon besteht.

7. Sandwich-Membran nach Anspruch 1, dadurch gekennzeichnet, daß die mikrostrukturierte Schicht aus einem Material besteht, welches Gebiete hoher Substratdurchlässigkeit wie Mikrokanäle oder Poren aufweist und eine hohe O₂-Permeabilität besitzt, die sich sowohl auf Gebiete hoher als auch auf die Gebiete niedriger Substratdurchlässigkeit bezieht.

8. Sandwich-Membran nach den Ansprüchen 1 und 7, dadurch gekennzeichnet, daß die Gebiete hoher Substratdurchlässigkeit 0.05 bis 20% der mikrostrukturierten Schicht ausmachen.

9. Sandwich-Membran nach den Ansprüchen 1,7 und 8, dadurch gekennzeichnet, daß die mikrostrukturierte Schicht eine Porendichte zwischen 1x10⁵ und 1x10⁹ P/cm² ,einen Porendurchmesser zwischen 0,01 und 0,8 µm und eine Dicke zwischen 2 und 40 µm hat.

10. Sandwich-Membran nach den Ansprüchen 1 und 7 bis 9, dadurch gekennzeichnet, daß die mikrostrukturierte Schicht bevorzugt aus einer oder mehreren Polycarbonat-, Polysiloxan-, Polyurethan-, Polysulfonat- oder Polyterephthalteilschichten besteht.

11. Sandwich-Membran nach Anspruch 1, dadurch gekennzeichnet, daß die Dialyseschicht/en aus Cellulose, Celluloseacetat oder Nylon bestehen.

12. Verwendung der Sandwich-Membran gemäß einem der Ansprüche 1 - 11, dadurch gekennzeichnet, daß die Membran an einen Sauerstoff detektierenden Transduktor gekoppelt und die Messung innerhalb eines bestimmten Zeitintervalls durchgeführt wird.

13. Verwendung nach Anspruch 12, dadurch gekennzeichnet, daß das Zeitintervall 1 bis 100, vorzugsweise 2-20 Sekunden, beträgt und daß die Registrierung des differenzierten Stromsignals die bevorzugte Detektionsmethode ist.

## Claims

1. A multilayer membrane for biosensors characterized by
- a layer of biomaterial
- separated from the sensor by a gas-selective layer
- and covered by a microstructured layer of inhomogeneous permeability to different substrates/cosubstrates in direction of the solution to be measured ;
the multilayer membrane optionally further comprising first and second dialysis layers disposed respectively between said gas selective layer and said biomaterial layer, and exterior of said microstructured layer, said second dialysis layer being adapted to contact the solution to be measured.

2. The multilayer membrane of claim 1, wherein the biomaterial layer is a polymeric material having bioparticles dispersed therein.

3. The multilayer membrane of claim 1 and 2, wherein the polymer is polyurethane, polyalginate, or gelatine.

4. The multilayer membrane of claim 1, 2, and 3, wherein the polymeric material with dispersed bioparticles is cross-linked by bi - or polyfunctional reagents.

5. The multilayer membrane of claim 1 and 2, wherein said bioparticles are preferable one enzyme or a multienzyme system, microorganisms or tissue cells.

6. The multilayer membrane of claim 1, wherein said gas selective layer is polyethylene, polypropylene, or polytetrafluoroethylene.

7. The multilayer membrane of claim 1, wherein the microstructured layer is a material having defined areas of high substrate permeation capability in form of microchannels or pores and a high oxygen permeability available in the areas of high and low substrate permeation characteristics as well.

8. The multilyer membrane of claim 1 and 7, wherein the areas of high substrate permeability comprise from about 0.05 % to about 20 % of the microstructured layer.

9. The multilayer membrane of claim 1, 7 and 8, wherein the microstructured layer is a porous layer, having a porosity of from about 1 x 10⁵ and 1 x 10⁹ p/cm², a pore diameter between about 0.01µm and about 0.8 µm and a thickness of between about 2 µm and about 40 µm.

10. The multilayer membrane of claim 1 and 7 to 9, wherein said microstructured layer comprises one or more polycarbonate, polysiloxane, polyurethane, polysulfonate, or polyterephthalate layers.

11. The multilayer membrane of claim 1, wherein said dialysis layer/s are of cellulose, cellulose acetate, or nylon.

12. Application of the multilayer membrane of claim 1 to 11, whereby the membrane is coupled to an oxygen-detecting transducer and the measurement is conducted within a defined time interval.

13. Application of claim 12, wherein said time intervall is from about 1 to about 100 s, 2 to 20 seconds by preference, and the registration of the differentiated current signal is the method of preference.

## Revendications

1. Membrane sandwiche pour biocapteurs, caractérisée par
- une couche de biomatériau qui du côté du capteur est dotée d'une couche sélective de gaz et qui
- du côté de la solution de mesure est couverte d'une couche microstructurée de perméabilité non-homogène pour les différents substrats /co-substrats,
- le cas échéant des couches de dialyse se trouvant respectivement entre la couche sélective de gaz et la couche de biomatériau, et sur la face externe de la couche microstructurée du côté de la solution de mesure.

2. Membrane sandwiche correspondant à celle décrite dans la revendication 1. et caractérisée par le fait que la couche de biomatériau est composée d'un matériau de polymère enrichi de bioparticules .

3. Membrane sandwiche correspondant à celle décrite dans les revendications 1. et 2., le matériau de polymère étant soi polyuréthane, soit polyalginate, soit gélatine.

4. Membrane sandwiche correspondant à celle décrite dans les revendications 1. à 3., le matériau de polymère étant lié aux bioparticules par des réactifs bi- ou polyfonctionnels.

5. Membrane sandwiche correspondant à celle décrite dans les revendications 1. et 2., caractérisée par le fait que les bioparticules sont composées de préférence d'une enzyme ou de plusieurs enzymes, de microorganismes ou de cellules de tissu.

6. Membrane sandwiche correspondant à celle décrite dans la revendication 1., caractérisée par le fait que la couche sélective de gaz est composée de polyéthylène, polypropylène ou de téflon.

7. Membrane sandwiche correspondant à celle décrite dans la revendication 1., caractérisée par le fait que la couche microstructurée est composée d'un matériau ayant des secteurs de haute perméabilité de substrate, tels microcanaux ou pores et ayant dans les secteurs de haute et de basse perméabilité de substrate une perméabilité élevée d'oxygène.

8. Membrane sandwiche correspondant à celle décrite dans les revendications 1. et 7, caractérisée par le fait que les secteurs de haute perméabilité de substrate s'élèvent à entre 0,05 et 20 % de la couche microstructurée.

9. Membrane sandwiche correspondant à celle décrite dans les revendications 1, 7 et 8, caractérisée par le fait que la couche microstructurée possède une concentration de pores entre 1x10⁵ et 1x10⁹ p/cm² , le diamètre des pores s'élevant à entre 0.01 et 0,8 µm et dont l'épaisseur se situe entre 2 et 40 µm.

10. Membrane sandwiche correspondant à celle décrite dans les revendications 1. et 7. à 9., caractérisée par le fait que la couche microstructurée est composée de préférence d'une ou de plusieurs couches partielles de polycarbonate, polysiloxane, de polyuréthane, de polysulfonate ou de polytéréphtalate.

11. Membrane sandwiche correspondant à celle décrite dans la revendication 1., caractérisée par le fait que la ou les couches de dialyse sont composées de cellulose, acétate de cellulose ou de nylon.

12. Emploi de la membrane sandwiche, selon les revendications de 1 à 11, caractérisé par le fait que la membrane est attachée à un transducteur détecteur d'oxygène et que la mesure est réalisée dans un intervalle de temps déterminé.

13. Emploi de la membrane sandwiche, selon la revendication 12, caractérisé par le fait que l'intervalle de temps monte à entre 1 et 100 secondes, de préférence à entre 2 et 20 secondes et que la méthode de détection préférée consiste dans l'enregistrement différentié du signal électrique.
